# EUROPEAN PATENT APPLICATION

(11) **EP 1 881 077 A1**
(43) Date of publication of application: **23.01.2008**
(21) Application number: 06117500.6
(22) Date of filing: 19.07.2006
(51) Int. Cl.: C12P 7/42, C12P 41/00

(54) **Process for the production of fluorine containing a-hydroxy carboxylic acids**

(71) Applicant: Solvay Organics GmbH, 30173 Hannover (DE)
(72) Inventor: Braun, Max, 30900, Wedemark (DE)
(74) Representative: Mross, Stefan P.M.

(57) **Abstract**

Process for the production of a fluorine containing α-hydroxy carboxylic acid by hydrolyzing a fluorine containing α-hydroxy nitrile in the presence of a nitrilase.

## Description

The present invention relates to a process for the production of fluorine containing α-hydroxy carboxylic acids. In particular, the present invention relates to a process for the production of fluorine containing α-hydroxy carboxylic acids by enzymatically catalyzed hydrolysis of the corresponding fluorine containing α-hydroxy nitriles. The fluorine containing α-hydroxy carboxylic acids can be obtained in enantiopure form.

(S)-3,3,3-trifluoro 2-hydroxy 2-methyl propionic acid is an important intermediate product for the manufacture of therapeutically useful amides (see e.g. EP-A-0 524 781).

It can for example be used to manufacture

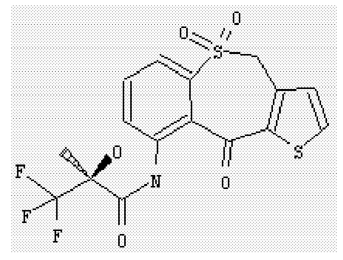

KW 7158, which is a candidate drug for the treatment of urinary incontinence.
The manufacture of the corresponding racemate and its separation into the enantiopure compounds is known, but the methods used for the separation are expensive. For example, EP-A-0 524 781 discloses a method for the manufacture of (S)-3,3,3-trifluoro 2-hydroxy 2-methyl propionic acid according to which the corresponding racemate is converted by means of (S)-(-)-α-methyl benzyl amine into the desired (S)-3,3,3-trifluoro 2-hydroxy 2-methyl propionic acid. High amounts of the amine are required which renders the method expensive.

DE 197 25 802 A1 discloses a method for the production of (S)- or (R)-3,3,3-trifluoro 2-hydroxy 2-methyl propionic acid by reacting first of all trifluoro acetic acid ester with a mineral acid to obtain 1,1,1-trifluoroacetone which is then reacted with cyanide to give the (S)- and (R)-3,3,3-trifluoro 2-hydroxy 2-methyl propionic acid nitrile. The nitrile is reacted with a C₁-C₄ alcohol to give a propionic acid ester which is then reacted in a further step by means of an esterase or lipase to (R)-3,3,3-trifluoro 2-hydroxy 2-methyl propionic acid. Upon biotransformation, in addition to the latter compound, the (S)-3,3,3-trifluoro 2-hydroxy 2-methyl propionic acid ester is also obtained which may then be hydrolyzed in a subsequent step to give (S)-3,3,3-trifluoro 2-hydroxy 2-methyl propionic acid.

The object of the present invention was therefore to provide an improved process for the production of fluorine containing α-hydroxy carboxylic acids. This process should allow in particular the production of enantiopure fluorine containing α-hydroxy carboxylic acids, in particular of (S)-1,1,1-trifluoro 2-hydroxy 2-methyl propionic acid.

Accordingly, the present invention provides a process for the production of fluorine containing α-hydroxy carboxylic acids by hydrolyzing fluorine containing α-hydroxy nitriles in the presence of a nitrilase.

In a preferred embodiment of the present invention the fluorine containing α-hydroxy nitrile is a α-hydroxy α-C₁-C₅-alkyl nitrile. More particularly, the fluorine containing α-hydroxy α-C₁-C₅-alkyl nitrile is (S)- and/or (R)-3,3,3-trifluoro 2-hydroxy 2-methyl propionic acid nitrile.

The nitrilase is suitably selected from the group consisting of NIT-102, NIT-104, NIT-106, NIT-107, and NIT-108. The nitrilase to be used in the process according to the present invention is preferably selected from the group consisting of NIT-104, NIT-106, NIT-107, and NIT-108, more preferably from the group consisting of NIT-104, NIT-106, and NIT-107. The most preferred nitrilase is NIT-106.

The nitrilases NIT-102, NIT-104, NIT-106, NIT-107, and NIT-108 are commercially available broad range nitrilases which may be obtained under these designations from Biocatalytics Inc., Pasadena, California, USA (Nitrilases : EC 3.5.5.1 CAS [9024-90-2]).

These nitrilases may catalyze the hydrolysis of nitriles to carboxylic acids at neutral pH and ambient temperature. There is in general no net consumption or production of protons, i.e., no change of pH.

The process according to the invention is generally carried out at a pH of from 6.5 to 8, preferably from 7.2 to 7.8. The reaction temperature is usually in the range of from 20 to 40°C, preferably in the range of from 25 to 35°C.

The process of the present invention can be carried out in various reaction media. The process of the invention is however preferably carried out in a buffer solution.

In a particular embodiment of the process according to the invention, the fluorine containing α-hydroxy nitrile is optically active and the hydrolysis is carried out under conditions under which the fluorine containing α-hydroxy nitrile is at least partially racemized. Such conditions are for example those mentioned here before.

In this embodiment, the racemization can be catalysed by the nitrilase, for example in particular NIT-106.

In the process of the invention, the nitrilases may be used in immobilized and lyophilized forms.

The term "enantiopure" as used herein refers in general to an enantiomeric excess (ee) - value of at least 90 %, preferably at least 95 % and most preferred at least 99 %.

The preferred starting compound in the process according to the present invention, 3,3,3-trifluoro 2-hydroxy 2-methyl propionic acid nitrile (as racemic mixture, R enantiomer and/or S enantiomer, respectively; in the following also simply referred to as "substrate") can be obtained by several methods. According to the present invention it is preferably obtained by reaction of 1,1,1-trifluoro acetone with a cyanide (see for example DE 197 25 802 A1). As the cyanide, HCN, NH4CN, NaCN or KCN may be suitably used. The reaction is preferably carried out in the presence of a mineral acid like for example sulfuric acid or hydrochloric acid. The overall reaction scheme for one embodiment can be represented as follows :

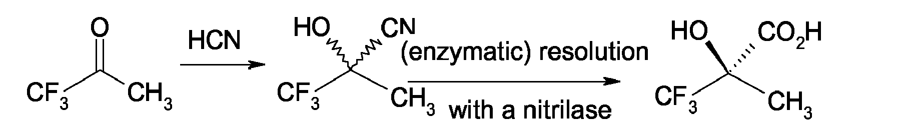

The process of the invention provides several advantages since the nitrilases used exhibit high enantio-, chemo- and regioselectivity. Hydrolysis may occur under mild reaction conditions which reduces the need for protection. Moreover, catalytic efficiencies are high. The waste is reduced and many organic solvents may be used.

The invention will be illustrated below in a non limitative way by the examples.

### EXAMPLES

In the Examples 1 to 8 and 10 to 14, racemic 3,3,3-trifluoro 2-hydroxy 2-methyl propionic acid nitrile was hydrolyzed at 30°C in the presence of one of the nitrilases NIT-101 to NIT-108 which had been obtained from Biocatalytics, Inc. from Pasadena, California, U.S.A. KPᵢ buffer (50 mM, pH 7.5, dithiothreitol DTT 2.0 mM, EDTA 1.0 mM) was used in each example.

### Examples 1 to 9

Racemic 3,3,3-trifluoro 2-hydroxy 2-methyl propionic acid nitrile (13.9 mg, 100 mM) was dissolved in KPi buffer (0.80 ml) at pH 7.5. To this solution, the corresponding nitrilase (2 mg) dissolved in KPi buffer (0.2 ml) was added. The concentration of the substrate in these experiments was thus 100 mM and the concentration of the nitrilase was 2 mg ml⁻¹.

The chemical composition of the solution obtained with each of the nitrilases was analyzed by gas chromatography. The sample preparation was performed as follows : Dichloromethane (0.4 ml) was added to the reaction mixture (1.0 ml). The mixture was then stirred vigorously for 30 seconds and thereafter centrifuged. The organic phase (0.2 ml) was converted with Bis(trimethylsilyl) trifluoracetamide (BSTFA) (0.05 ml). After 15 min gas chromatography was carried out.

The determination of the conversion rate (see Table 1) as well as of the enantiomeric excess (see Examples 10 to 14; Table 2) was determined by gas chromatography as follows :

| | |
|---|---|
| Column: | CP-Chirasil-DEX CB, |
| Flow : | Helium (const. 17 psi [1.17 bar]), |
| Injector temperature : | 250°C, |
| Split : | 1 : 150, |
| Detector Temperature : 275°C (FID) | |

Temperature program :
t = 0 min: 70°C, hold 15 min; t = 15 min: rate 20°C min⁻¹; t = 19.5 min: 160°C, hold 5.5 min.
Rₜ (substrate, first enantiomer, TMS-derivative) : 2.56 min,
Rₜ (substrate, second enantiomer, TMS-derivative) : 2.61 min,
Rₜ (product, first enantiomer, TMS-derivative) : 8.44 min,
Rₜ (product, second enantiomer, TMS-derivative) : 8.79 min,
Rₜ (by-product) : 2.81 min (probably the bis-TMS-derivative).

The conversion rates of all reactions performed under the above mentioned condition using the nitrilases NIT-101 to NIT-108 after 5 min, 5 h, 24 h and 48 h are summarized in Table 1. Conversion in terms of a decrease of the substrate concentration was detected for all enzymatic reactions (Examples 1 to 8). A decrease in the substrate conversion was however also observed for Example 9 in which no nitrilase had been used. Accordingly, the substrate is unstable in water. High conversion was observed in Examples 2, 4, 6, 7 and 8, respectively, in which the enzymes NIT-102, NIT-104, NIT-106, NIT-107 and NIT-108 were employed. Especially, the enzymes NIT-104 and NIT-107 showed an evident increase of the conversion (see Examples 4 and 7, respectively) in that the substrate was converted within 5 hours by more than 90 %. In the remaining examples, at least an increase of the initial conversion rate after 5 hours was observed.

**Table 1**

| Examples | Commercially available nitrilase | Conversion of the nitrile [%] | | | |
|---|---|---|---|---|---|
| | | After 5 min | After 5h | After 24 h | After 48 h |
| 1 | NIT-101 | < 1 | 10.1 | 31.0 | 32.5 |
| 2 | NIT-102 | < 1 | 43.1 | 53.6 | 52.2 |
| 3 | NIT-103 | < 1 | 11.1 | 29.8 | 33.0 |
| 4 | NIT-104 | 1.6 | 93.6 | > 99.5 | > 99.5 |
| 5 | NIT-105 | < 1 | 16.6 | 32.7 | 28.8 |
| 6 | NIT-106 | < 1 | 72.8 | > 99.5 | > 99.5 |
| 7 | NIT-107 | < 1 | 95.6 | > 99.5 | > 99.5 |
| 8 | NIT-108 | < 1 | 44.4 | 91.2 | 97.1 |
| 9 * | No enzyme | < 1 | < 1 | 26.1 | 33.0 |

| | | | | | |
|---|---|---|---|---|---|
| *Example not according to the invention. | | | | | |

In none of the performed reactions, one of the enantiomers of the substrate was converted faster than the other to the carboxylic acid. Independent of the reaction time, the enantiomeric excesses of the substrate (including the reactions containing no enzyme) were always lower than 10 %. This phenomenon may be explained for example by racemization of the substrate under the reaction condition employed. Such racemization is advantageous inasmuch as it allows for access to high chemical yield of desired product.

### Examples 10 to 14

In order to elucidate the composition of the reaction mixture after complete conversion, the reactions with the nitrilases NIT-102, NIT-104, NIT-106, NIT-107 and NIT-108, respectively, were repeated on a semi-preparative scale (5.0 ml) at 30°C in KPᵢ buffer (50 mM, pH 7.5, DTT 2.0 mM, EDTA 1.0 mM) as solvent as follows.

The substrate (racemic 3,3,3-trifluoro 2-hydroxy 2-methyl propionic acid nitrile; 70.0 mg, 100 mM) was dissolved in KPᵢ buffer (4.00 ml). To this solution, the corresponding nitrilase (10 mg) dissolved in KPi buffer (1.0 ml) was added. The concentration of the substrate in these experiments was thus 100 mM and the concentration of the nitrilase was 2 mg ml⁻¹. After the conversion was complete, the reaction mixture was acidified with HCl (pH 1.0). The reaction mixture was then extracted with methyl tert.-butyl ether (MtBE) trice. The combined organic phases were dried with Na₂SO₄ and the solvent removed under reduced pressure.

The chemical composition of the solution obtained with each of the nitrilases was analyzed by gas chromatography. Sample preparation was performed as follows. The crude product (5 mg) was dissolved in MtBE (0.2 ml) and BSTFA (0.05 ml) was then added. After 15 min, gas chromatographic analysis was carried out. The results are shown in Table 2.

The isolated crude product was analyzed by GC under the conditions described above for Examples 1 to 9. It appeared that the product was generated asymmetrically in all reactions. With the exception of Example 10, wherein a high conversion of substrate was obtained with relatively low enantiomeric access, the obtained enantiomeric excesses were moderate to high. Best results were obtained using NIT-106 (ee > 99 %, high isolated yield).

For the crude product isolated from the reaction catalyzed by NIT-106, the melting point and the specific optical rotation was determined :
Melting point = 102.5 -104°C
Specific optical rotation = [α]_{D}²⁰ = -15.3° ± 2

The melting point confirms that the desired product, 3,3,3-trifluoro 2-hydroxy 2-methyl propionic acid, was obtained. The value for the specific optical rotation indicates that the obtained compound has [S] configuration. This could also be confirmed by comparison of the GC data of this enzymatically produced compound with those obtained for commercially available [S]-3,3,3-trifluoro 2-hydroxy 2-methyl propionic acid.

NIT 106 gave an enantiomeric excess value (ee-value) for the S enantiomer of > 99 %. Surprisingly, NIT 106 could moreover catalyze the racemisation of the remaining R-enantiomer. NIT-106 was thus an ideal nitrilase for producing selectively the S enantiomer (asymmetric process to the S enantiomer).

**Table 2**

| Examples | Nitrilase | Reaction time [h] | Amount of isolated crude product [mg(%)] | Composition of the crude product | | | |
|---|---|---|---|---|---|---|---|
| | | | | Substrate | | Product | |
| | | | | Amount [%] | Ee [%] | Amount [%] | ee [%] |
| 10 | NIT-102 | 24 | Not determined | < 0.5 | - | > 99.5 | low |
| 11 | NIT-104 | 5 | 10.0 (14) | 0.8 | > 90 | 99.2 | 50 |
| 12 | NIT-106 | 24 | 48.1 (69) | < 0.5 | - | > 99.5 | > 99 |
| 13 | NIT-107 | 5 | 9.2 (13) | 0.6 | > 90 | 99.4 | 77 |
| 14 | NIT-108 | 24 | 13.4 (19) | < 0.5 | - | > 99.5 | High^{a} |

| | | | | | | | |
|---|---|---|---|---|---|---|---|
| ^{a}: Instead of the mono-trimethylsilyl derivative, the bis-trimethylsilyl derivative of the product was detected, for which a base-line separation could not be realized. | | | | | | | |

## Claims

1. Process for the production of a fluorine containing α-hydroxy carboxylic acid which comprises hydrolyzing a fluorine containing α-hydroxy nitrile in the presence of a nitrilase.

2. Process according to claim 1, wherein the fluorine containing α-hydroxy nitrile is a α-hydroxy α-C₁-C₅-alkyl nitrile.

3. Process according to claim 2, wherein the fluorine containing α-hydroxy α-C₁-C₅-alkyl nitrile is (S)- and/or (R)-3,3,3-trifluoro 2-hydroxy 2-methyl propionic acid nitrile.

4. Process according to any of claims 1 to 3, wherein the nitrilase is selected from the group consisting of NIT-102, NIT-104, NIT-106, NIT-107, and NIT-108.

5. Process according to claim 4, wherein the nitrilase is selected preferably from NIT-104, NIT-106 and NIT-107.

6. Process according to claim 5, wherein the nitrilase is NIT-106.

7. Process according to any of claims 1 to 6, wherein the hydrolysis is carried out at a temperature of from 20 to 40°C.

8. Process according to any of claims 1 to 7, wherein the hydrolysis is carried out at a pH of from 6.5 to 8.

9. Process according to anyone of claims 1 to 8, wherein the fluorine containing α-hydroxy nitrile is optically active and the hydrolysis is carried out under conditions under which the fluorine containing α-hydroxy nitrile is at least partially racemized.

10. Process according to claim 9, wherein the racemization is catalysed by the nitrilase.
